# EUROPÄISCHE PATENTSCHRIFT

(11) **EP 3 791 782 B1**
(45) Veröffentlichungstag und Bekanntmachung des Hinweises auf die Patenterteilung: **02.08.2023**
(21) Anmeldenummer: 19196443.6
(22) Anmeldetag: 10.09.2019
(51) Int. Cl.: A61B 5/287

(54) **KATHETER MIT FLEXIBLER LEITERPLATTE UND FERTIGUNGSVERFAHREN FÜR FLEXIBLE LEITERPLATTE**
CATHETER WITH FLEXIBLE CIRCUIT BOARD AND METHOD OF MANUFACTURING FLEXIBLE CIRCUIT BOARD
CATHÉTER POURVU DE CARTE DE CIRCUIT IMPRIMÉ FLEXIBLE ET PROCÉDÉ DE FABRICATION DE CARTE DE CIRCUIT IMPRIMÉ FLEXIBLE

(43) Veröffentlichungstag der Anmeldung: 17.03.2021
(73) Patentinhaber: Freudenberg SE, 69469 Weinheim (DE)
(72) Erfinder: Schmied, Benno, 67071 Ludwigshafen (DE)

(56) Entgegenhaltungen:
- WO-A1-2016/171597
- JP-A- 2006 244 968
- US-A1- 2006 244 177
- US-A1- 2007 219 551
- US-A1- 2012 172 696
- US-A1- 2016 113 729
- US-A1- 2019 175 056

## Beschreibung

Die Erfindung betrifft einen Katheter gemäß dem Oberbegriff von Anspruch 1 mit einer flexiblen Leiterplatte und ein Verfahren zur Fertigung einer flexiblen Leiterplatte gemäß Anspruch 4.

### Stand der Technik

Aus dem Stand der Technik sind unterschiedlichste Bauformen von Kathetern bekannt, welche ihrer medizinischen Anwendung entsprechend aufgebaut sind. Darunter sind auch elektrodentragende Katheter wie sie beispielsweise in der DE 694 01 562 T2 beschrieben sind. Auch bekannt sind sogenannte Mappingkatheter, beispielsweise zur 3-dimensionalen Erfassung der Anatomie einer Herzkammer, welche eine hohe Anzahl an Elektroden aufweisen müssen.

Die US 2007/219551 A1 offenbart einen Katheter mit den Merkmalen des Oberbegriffs von Anspruch 1.

Die US 2006/244177 A1 offenbart eine flexible Leiterplatte für einen Katheter. Die flexible Leiterplatte weist ein bandartiges Mäandermuster mit mehreren linearen Teilen auf, die etwa parallel zueinander angeordnet sind, und einen Falzrand, der mit einem Ende in der Längsrichtung zweier benachbarter linearer Teile der mehreren linearen Teile verbunden ist, Diese Leiterplatte lässt sich in ein einzelnes lineares Produkt mit einer Gesamtlänge von nicht weniger als 300 mm verwandeln, wenn der Falzrand etwa in der Mitte davon doppelt gefaltet ist und am Falzrand einer der beiden linearen Teile, die mit dem Falzrand verbunden sind, in der entgegengesetzten Richtung um 180 Grad zurückgefaltet wird.

Bei den aus dem Stand der Technik bekannten Kathetern entstehen hohe Produktionskosten, weil die zu den Elektroden führenden Leiterbahnen aufwändig im Katheterrohr untergebracht werden müssen. Weiter problematisch ist, dass sich auf Grund der verwendeten Materialien eine Beschränkung bzgl. des Biegeradius des Katheters ergeben kann und damit eine Einschränkung in dessen Einsatz.

### Aufgabenstellung

Aufgabe der vorliegenden Erfindung ist es, einen kostengünstigen und gleichzeitig flexiblen Katheter zu schaffen und die Nachteile des Standes der Technik zumindest teilweise zu beheben. Weitere Aufgabe ist es, ein kostengünstiges Fertigungsverfahren für flexible Leiterplatten zu beschreiben, welche in einem solchen Katheter eingesetzt werden können.

### Technische Lösung

Gelöst wird diese Aufgabe durch einen Katheter mit den Merkmalen von Anspruch 1.

Erfindungsgemäß wurde als vorteilhaft erkannt eine flexible Leiterplatte mit Leiterbahnen zu verwenden:
Der Katheter hat die Form eines länglichen, hohlen und flexiblen Rohres, welches auch als Schlauch bezeichnet werden könnte. Es handelt sich um einen Hohlkatheter. Der Katheter besitzt mindestens eine Elektrode zur Durchführung einer Diagnose, beispielsweise einer Messung wie einer elektrophysiologischen Untersuchung, oder zur Durchführung einer Therapie, beispielsweise einer Operation wie einer Katheterablation, jeweils im menschlichen Körper. Die mindestens eine Elektrode ist an einem Endbereich des Rohres angeordnet und am anderen Endbereich des Rohres ist ein elektrischer Anschluss angebracht zum elektrischen Verbinden des Katheters mit einer Steuereinrichtung, sodass eine stromleitende und datenübertragungstechnische Verbindung zwischen der mindestens eine Elektrode und dem elektrischen Anschluss besteht.

Erfindungsgemäß weist die Leiterplatte die Form eines langen Bandes auf. Dies hat den Vorteil, dass dann eine einzige Leiterplatte genügen kann, um die Verbindung von Elektrode und elektrischen Anschluss über die ganze Länge des Rohres herzustellen.

Erfindungsgemäß ist das Band plissiert, d. h. mit einer Zickzack Faltung versehen.

Eine derartige verformte Leiterplatte kann Biegebewegungen des Rohres besonders gut mitmachen, toleriert selbst enge Biegeradien, und es wird verhindert, dass auf die Leiterplatte bei der Biegebewegung hohe Dehnungen wirken und die Leiterbahnen brechen könnten.

In vorteilhafter Weise sind die mindestens eine Elektrode und der elektrische Anschluss mittels Leiterbahnen auf mindestens einer flexiblen Leiterplatte elektrisch leitend miteinander verbunden. Die flexible Leiterplatte ist insbesondere filmartig und biegeschlaff aber nicht notwendigerweise dehnbar. Als Material kann beispielsweise Polyimid (Pl) eingesetzt werden. Die Leiterbahnen können insbesondere aus Kupfer oder aus Kupferlegierungen gefertigt sein. Gemäß der Erfindung ist die flexible Leiterplatte in dem Rohr angeordnet, d. h. innerhalb des hohlen Rohres.

Ein derartiger Katheter ist besonders kostengünstig in seiner Fertigung, da keine einzelnen Stromleiter verlegt werden müssen. Durch die Verwendung einer flexiblen Leiterplatte im Inneren des Rohres ist der Katheter auch sehr flexibel.

Besonders vorteilhaft erscheint es, wenn das Band als Band konstanter Breite schneckenförmig aus einer flexiblen Leiterplatte herausgetrennt ist. Eine derartige flexible Leiterplatte ist besonders kostengünstig in ihrer Herstellung. Näheres geht untenstehend aus der Beschreibung des erfindungsgemäßen Fertigungsverfahrens hervor.

Es hat sich als besonders vorteilhaft erwiesen, wenn das Band onduliert ist, d.h. in seiner Längsachsrichtung spiralförmig gewunden ist.

Erfolgt zusätzlich ein Verdrehen der Leiterplatte um die Längsachse des Katheters, so werden die Freiheitsgrade der Leiterplatte weiter erhöht und eine Beschädigung der Leiterplatte noch besser verhindert.

Die Erfindung betrifft auch ein Verfahren zur Fertigung einer flexiblen Leiterplatte in Form eines langen Bandes, insbesondere für einen Katheter wie obenstehend beschrieben, mit folgenden Schritten:
a. Bereitstellen einer flexiblen Trägerplatte.
b. Aufbringen von Leiterbahnen auf die flexiblen Trägerplatte zur Herstellung einer flächigen Leiterplatte.
c. Heraustrennen eines Bandes aus der flächigen Leiterplatte, wobei die Trennlinie die Form einer schneckenförmigen Spirale aufweist, d. h einer Archimedischen Spirale. In einer Alternativen Verfahrensvariante kann die Trennlinie eine U-Form aufweisen, mit zwei Schenkeln und einer die Schenkel verbindenden Krümmung.
   Als zusätzlicher fakultativer Schritt kann ein Strecken des Bandes durch Aufbringen von entgegengesetzten Zugkräften an den Enden des Bandes erfolgen. Dadurch wird die Spirale zu einem langen, ondulierten Band aufgezogen.
d. Plissieren, d.h. Zickzack-Falten des Bandes.

Ein derartiges Fertigungsverfahren zur Herstellung einer flexiblen Leiterplatte ist besonders kostengünstig, da es zum einen materialsparend ist und zum anderen schnell ist, weil eine Einrichtung zum Aufbringen der Leiterbahnen und ein Werkzeug zum Heraustrennen nur eine vergleichsweise kleine Fläche erreichen können muss, also nur kleine Relativbewegungen realisiert werden müssen.

Weiter vorteilhaft ist, dass sich eine Ondulierung der bandförmigen Leiterplatte automatisch ergibt und damit eine gute Flexibilität der Leiterplatte erreicht wird.

Es hat sich als besonders vorteilhaft erwiesen, wenn die Spirale einen konstanten Windungsabstand aufweist, derart dass ein Band konstanter Breite entsteht. Bei der Spirale handelt es sich dann um eine Kreisevolvente.

In vorteilhafter Weiterbildung des erfindungsgemäßen Verfahrens erfolgt das Trennen durch Stanzen oder Schneiden, insbesondere Brennschneiden oder Strahlschneiden, wie beispielsweise Wasserstrahlschneiden oder Laserstrahlschneiden.

Die Erfindung betrifft auch die Verwendung eines wie obenstehend beschriebenen Katheters als Herzkatheter, welcher in der Diagnose oder Therapie des menschlichen Herzens zum Einsatz gelangt.

Die beschriebene Erfindung und die beschriebenen vorteilhaften Weiterbildungen der Erfindung stellen auch in Kombination miteinander - soweit dies technisch sinnvoll ist - vorteilhafte Weiterbildungen der Erfindung dar.

Hinsichtlich weiterer Vorteile und in konstruktiver und funktioneller Hinsicht vorteilhafter Ausgestaltungen der Erfindung wird auf die Unteransprüche sowie die Beschreibung von Ausführungsbeispielen unter Bezugnahme auf die beiliegenden Figuren verwiesen.

### Ausführungsbeispiel

Die Erfindung soll an Hand beigefügter Figuren noch näher erläutert werden. Einander entsprechende Elemente und Bauteile sind in den Figuren mit gleichen Bezugszeichen versehen. Zugunsten einer besseren Übersichtlichkeit der Figuren wurde auf eine maßstabsgetreue Darstellung verzichtet.

Es zeigen in schematischer Darstellung
- Fig. 1: einen erfindungsgemäßen Katheter
- Fig. 2a: eine materialsparende und kostengünstige Fertigung einer Leiterplatte
- Fig. 2b: eine alternative materialsparende und kostengünstige Fertigung einer Leiterplatte
- Fig. 3: eine Leiterplatte in ondulierter Form
- Fig. 4: eine ondulierte Leiterplatte innerhalb eines Rohres
- Fig. 5: den Vorgang des Plissierens einer Leiterplatte

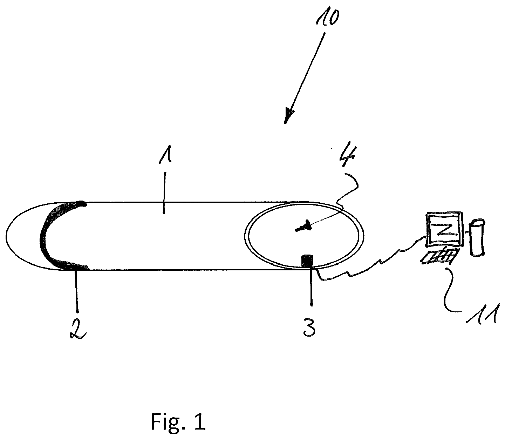

Fig. 1 zeigt einen erfindungsgemäßen Katheter 10, welcher die Form eines länglichen, flexiblen Rohres 1 besitzt. An seinem einen Endbereich ist mindestens eine Elektrode 2 angeordnet. An seinem anderen Endbereich ist ein elektrischer Anschluss 3 angebracht. Die mindestens eine Elektrode 2 ist über Leiterbahnen 5 auf einer elastisch dehnbaren Leiterplatte 4 (hier nicht näher dargestellt) innerhalb des Rohres 1 mit dem elektrischen Anschluss 3 datenübertragungstechnisch und stromleitend verbunden. An den Anschluss 3 ist eine Steuereinrichtung 11 angeschlossen, welche zur Steuerung des Katheters 10 dient.

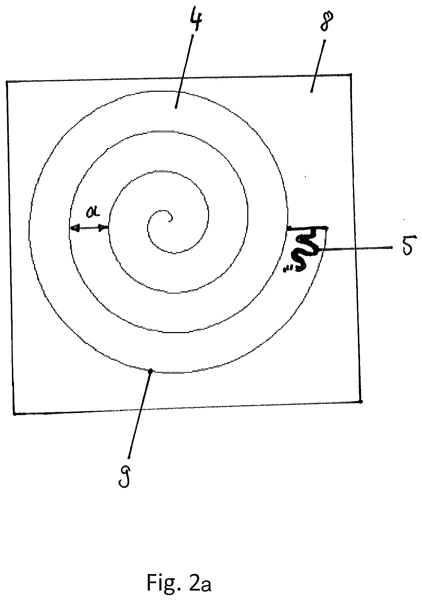

Fig. 2a zeigt eine materialsparende und kostengünstige Möglichkeit zur Fertigung einer Leiterplatte 4. Es erfolgt zuerst ein Bereitstellen einer flexiblen Trägerplatte 8. Danach erfolgt ein Aufbringen von Leiterbahnen 5 auf die flexiblen Trägerplatte 8 zur Herstellung einer flächigen Leiterplatte 4. Die Leiterbahnen 5 sind in der Figur nur angedeutet. Sodann erfolgt ein Heraustrennen eines Bandes aus der flächigen Leiterplatte 4, wobei die Trennlinie 9 die Form einer schneckenförmigen Spirale aufweist, d. h einer Archimedischen Spirale. Der Windungsabstand a der Spirale ist konstant, derart, dass ein Band konstanter Breite entsteht.

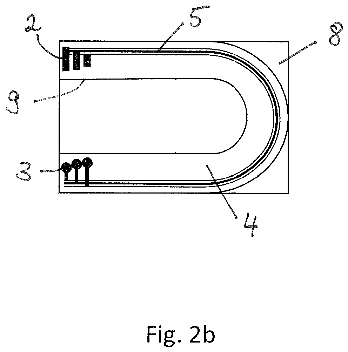

Fig. 2b zeigt eine alternative materialsparende und kostengünstige Möglichkeit zur Fertigung einer Leiterplatte 4, welche besonders vorteilhaft ist, wenn mehrere Elektroden an einem Katheder vorgesehen sind. Es erfolgt zuerst ein Bereitstellen einer flexiblen Trägerplatte 8. Danach erfolgt ein Aufbringen von Leiterbahnen 5 auf die flexiblen Trägerplatte 8 zur Herstellung einer flächigen Leiterplatte 4. Die Leiterbahnen 5 sind in der Figur nur als Linien angedeutet, obwohl sie in der Praxis bevorzugt eine Mäanderform aufweisen. Auch die Elektroden 2 und elektrischen Anschlüsse 3 können aufgebracht werden. Sodann erfolgt ein Heraustrennen eines Bandes aus der flächigen Leiterplatte 4, wobei die Trennlinie 9 die Form einer Aneinanderreihung einer Geraden, eines Halbkreises und nochmals einer Geraden aufweist, also einer U-Form mit zwei Schenkeln und einer die Schenkel verbindenden Krümmung. Elektroden 2 und elektrische Anschlüsse 3 liegen in den geraden Bereichen und können so einfacher hergestellt werden.

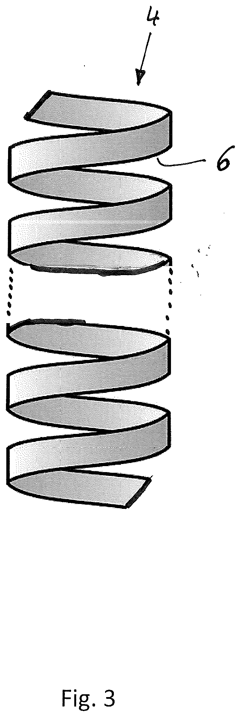

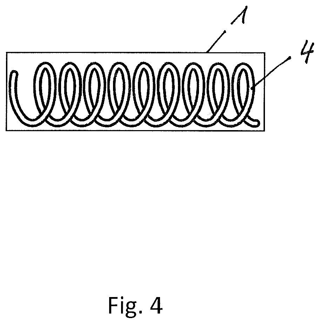

Fig. 3 zeigt eine Leiterplatte 4 in Form eines langen Bandes mit Ondulierung 6, welche sich durch das in Fig. 2 gezeigte Fertigungsverfahren ergibt. Eine derartig ondulierte Leiterplatte 4 kann zur Schaffung eines Katheters 10 innerhalb eines Rohres 1 positioniert sein, wie in Fig. 4 gezeigt.

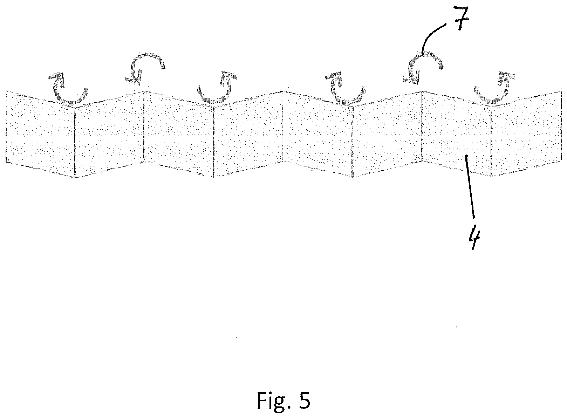

Fig. 5 zeigt den Vorgang des Plissierens einer bandförmigen Leiterplatte 4. Das Plissieren, d.h. Erzeugen eines Plissee 7 erfolgt durch zickzackartiges Falten der Leiterplatte 4.

### Bezugszeichenliste

- 1: Rohr
- 2: Elektrode
- 3: Anschluss
- 4: Leiterplatte
- 5: Position Leiterbahn
- 6: Ondulierung
- 7: Plissee
- 8: Trägerplatte
- 9: Trennlinie
- 10: Katheter
- 11: Steuereinrichtung

- a: Windungsabstand

## Patentansprüche

1. Katheter (10) in Form eines länglichen, hohlen und flexiblen Rohres (1) mit mindestens einer Elektrode (2) zur Durchführung einer Diagnose oder Therapie im menschlichen Körper, wobei die mindestens eine Elektrode (2) an einem Endbereich des Rohres (1) angeordnet ist und am anderen Endbereich des Rohres (1) ein elektrischer Anschluss (3) angebracht ist, zum elektrischen Verbinden des Katheters (10) mit einer Steuereinrichtung (11), wobei die mindestens eine Elektrode (2) und der elektrische Anschluss (3) mittels Leiterbahnen (5) auf einer flexiblen Leiterplatte (4) elektrische leitend miteinander verbunden sind und, wobei die flexible Leiterplatte (4) in dem Rohr (1) angeordnet ist,
**dadurch gekennzeichnet, dass**
die Leiterplatte (4) die Form eines langen plissierten Bandes aufweist.

2. Katheter nach Anspruch 1, **dadurch gekennzeichnet, dass**
das Band als Band konstanter Breite schneckenförmig aus einer flexiblen Leiterplatte (4) herausgetrennt ist.

3. Katheter nach einem der Ansprüche 1 oder 2, **dadurch gekennzeichnet, dass**
das Band onduliert ist.

4. Verfahren zur Fertigung einer flexiblen Leiterplatte (4) in Form eines langen Bandes, insbesondere für einen Katheter (10) nach einem der vorangehenden Ansprüche,
mit folgenden Schritten:
a. Bereitstellen einer flexiblen Trägerplatte (8),
b. Aufbringen von Leiterbahnen (5) auf die flexiblen Trägerplatte (8) zur Herstellung einer flächigen Leiterplatte (4),
c. Heraustrennen eines Bandes aus der flächigen Leiterplatte (4), wobei die Trennlinie (9) die Form einer schneckenförmigen Spirale oder eine U-Form aufweist
d. Plissieren (7) des Bandes.

5. Verfahren nach Anspruch 4, **dadurch gekennzeichnet, dass**
die Spirale einen konstanten Windungsabstand (a) aufweist.

6. Verfahren nach einem der Ansprüche 4 oder 5 **dadurch gekennzeichnet, dass**
das Heraustrennen durch Stanzen oder Schneiden erfolgt.

## Claims

1. Catheter (10) in the form of an elongate, hollow and flexible tube (1) having at least one electrode (2) for carrying out a diagnosis or therapy in the human body, the at least one electrode (2) being arranged at an end region of the tube (1), and an electrical terminal (3) being attached at the other end region of the tube (1) in order to electrically connect the catheter (10) to a control device (11), the at least one electrode (2) and the electrical terminal (3) being connected to each other in an electrically conductive manner by means of conductor tracks (5) on a flexible printed circuit board (4), and the flexible printed circuit board (4) being arranged in the tube (1), **characterized in that** the printed circuit board (4) has the shape of a long pleated band.

2. Catheter according to Claim 1, **characterized in that** the band is separated as a band of constant width in a helical shape from a flexible printed circuit board (4).

3. Catheter according to one of Claims 1 or 2, **characterized in that** the band undulates.

4. Method for manufacturing a flexible printed circuit board (4) in the form of a long band, in particular for a catheter (10) according to one of the preceding claims, the method having the following steps:
a. making available a flexible carrier plate (8),
b. applying conductor tracks (5) to the flexible carrier plate (8) so as to produce a planar printed circuit board (4),
c. separating a band from the planar printed circuit board (4), the separation line (9) having the shape of a helical spiral or a U-shape,
d. pleating (7) the band.

5. Method according to Claim 4, **characterized in that** the spiral has a constant turn spacing (a).

6. Method according to one of Claims 4 or 5, **characterized in that** the separation is effected by punching or cutting.

## Revendications

1. Cathéter (10) sous la forme d'un tube (1) allongé, creux et flexible comportant au moins une électrode (2) pour effectuer un diagnostic ou une thérapie dans le corps humain, l'au moins une électrode (2) étant agencée au niveau d'une région d'extrémité du tube (1) et une borne électrique (3) étant fixée au niveau de l'autre région d'extrémité du tube (1), pour la connexion électrique du cathéter (10) à un dispositif de commande (11), l'au moins une électrode (2) et la borne électrique (3) étant connectées électriquement l'une à l'autre au moyen de pistes conductrices (5) sur une carte de circuit imprimé (4) flexible, et la carte de circuit imprimé (4) flexible étant agencée dans le tube (1),
**caractérisé en ce que**
la carte de circuit imprimé (4) présente la forme d'une longue bande plissée.

2. Cathéter selon la revendication 1, **caractérisé en ce que**
la bande est séparée d'une carte de circuit imprimé (4) flexible de manière hélicoïdale sous forme de bande de largeur constante.

3. Cathéter selon l'une quelconque des revendications 1 ou 2, **caractérisé en ce que**
la bande est ondulée.

4. Procédé de fabrication d'une carte de circuit imprimé (4) flexible sous la forme d'une longue bande, en particulier pour un cathéter (10) selon l'une des revendications précédentes,
comportant les étapes suivantes :
a. fourniture d'une plaque de support (8) flexible,
b. application de pistes conductrices (5) sur la plaque de support (8) flexible pour la production d'une carte de circuit imprimé (4) plane,
c. séparation d'une bande de la carte de circuit imprimé (4) plane, la ligne de séparation (9) présentant la forme d'une spirale hélicoïdale ou une forme en U,
d. plissage (7) de la bande.

5. Procédé selon la revendication 4, **caractérisé en ce que**
la spirale présente un pas (a) constant.

6. Procédé selon l'une quelconque des revendications 4 ou 5, **caractérisé en ce que**
la séparation s'effectue par estampage ou découpage.
